(19) Europäisches Patentamt
European Patent Office
Office européen des brevets

(11) EP 0 702 026 A2

(12) EUROPÄISCHE PATENTANMELDUNG

(43) Veröffentlichungstag: **20.03.1996 Patentblatt 1996/12**

(21) Anmeldenummer: **95114107.6**

(22) Anmeldetag: **08.09.1995**

(51) Int. Cl.[6]: **C07J 9/00**, A61K 31/575, C07J 17/00, C07J 41/00, C07J 51/00

(84) Benannte Vertragsstaaten:
**AT BE CH DE DK ES FR GB GR IE IT LI LU NL PT SE**

(30) Priorität: **14.09.1994 DE 4432708**

(71) Anmelder: **HOECHST AKTIENGESELLSCHAFT
D-65929 Frankfurt am Main (DE)**

(72) Erfinder:
- **Wess, Günther, Dr.
D-63526 Erlensee (DE)**
- **Enhsen, Alfons, Dr.
D-64572 Büttelborn (DE)**
- **Kramer, Werner, Dr. Dr.
D-55130 Mainz (DE)**
- **Bock, Klaus
D-65795 Hattersheim (DE)**

# (54) Modifizierte Gallensäuren, Verfahren zu ihrer Herstellung und ihre Verwendung

(57) Die Erfindung betrifft Linker-modifizierte Gallensäurederivate der Formel I

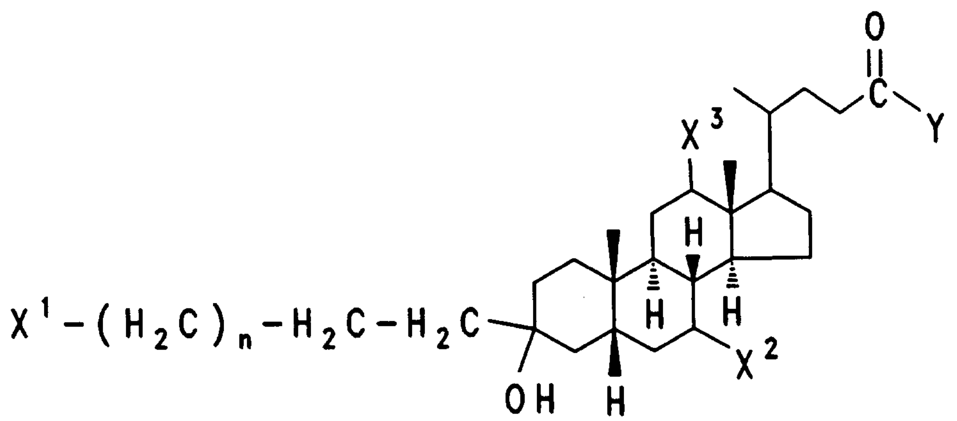

I

wobei die Reste $X^1$, $X^2$, $X^3$ und Y und n die in den Ansprüchen angegebenen Bedeutungen haben, und Verfahren zu ihrer Herstellung. Die erfindungsgemäßen Verbindungen sind therapeutisch wirksam und eignen sich somit als Arzneimittel, insbesondere als Hypolipidaemika. Die Erfindung betrifft weiterhin die Verwendung der erfindungsgemäßen Gallensäurederivate als Arzneimittel.

EP 0 702 026 A2

## Beschreibung

Gallensäuren mit Linkerstrukturen in den steroidalen Positionen C-3, C-7 und C-12 sind für pharmazeutische Anwendungen von Interesse. So gelingt es, durch Kopplung von Wirkstoffen an Gallensäuren über geeignete Linker eine leberspezifische Wirkstoffaufnahme und dadurch eine pharmakologische Wirkung zu erreichen bzw. die Resorption von schlecht resorbierbaren Pharmaka wie z.B. Peptiden zu verbessern (siehe z.B. Kramer et al., J. Biol. Chem., 267: 18598-18604, 1992; Kramer et al., J. Biol. Chem., 269: 1-7, 1994; Europäische Patentanmeldung EP-A-0 417 725 (entspricht US-Patentanmeldung Serial No. 08/208,192)). Verknüpfung von Gallensäuren über Linkerstrukturen führt zu Hemmstoffen der Gallensäureresorption (siehe z.B. Wess et al., J. Med. Chem., 37: 873, 1994; Europäische Patentanmeldungen EP-A-0 573 848 (entspricht US-Patentanmeldung Serial No. 08/074,753) und EP-A-0 489 423).

Im Stand der Technik wird beschrieben, daß für diese Anwendungen modifizierte Gallensäuren verwendet werden können, die keine 3-OH Gruppe an dem den Linker tragenden Kohlenstoffatom besitzen. Aufgrund fehlender Ausgangsprodukte zur Herstellung von Verbindungen mit variablen Linkern und Funktionalitäten an der Endposition des Linkers sowie gleichzeitiger Anwesenheit einer OH-Gruppe an der Verknüpfungsstelle des Linkers mit dem Gallensäureteil (3-C) sind diese Verbindungen bisher unbekannt.

Es wurde gefunden, daß die erfindungsgemäßen Verbindungen der Formel I durch direkte Verknüpfung eines geeigneten Acetylenderivates mit einem geeigneten Gallensäureketon zugänglich sind. Es wurde weiterhin gefunden, daß an diesen primären Additionsprodukten und ihren Folgeprodukten eine Reihe von chemischen Umwandlungen möglich sind, ohne daß die Anwesenheit der OH-Gruppe an der Verknüpfungsstelle störende Nebenreaktionen verursacht.

Die Erfindung betrifft Linker-modifizierte Gallensäurederivate der Formel I,

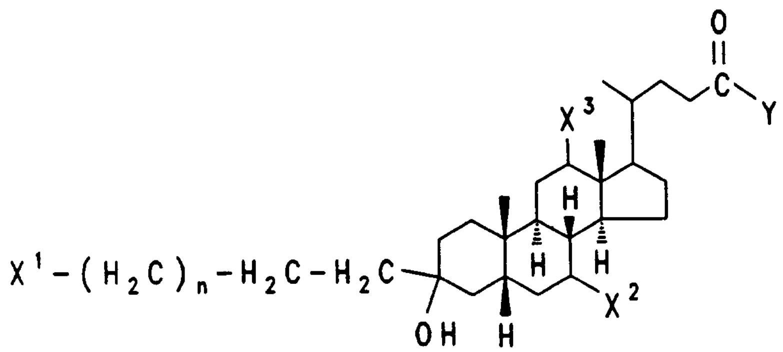

I

wobei

X[1]
- $CH_2OH$,
- $CH_2NH_2$,
- $CH_2OSil$, wobei Sil eine Silylschutzgruppe der Formel $SiMe_3$, $SiMe_2t$-Bu oder $SiPh_2t$-Bu bedeutet,
- $CH_2OTHP$,
- $CH_2OCH_2Ph$, wobei Phenyl p-Methoxy substituiert sein kann,
- $CH_2O$-Trityl,
- $CH_2O$-Acyl, wobei Acyl für einen geradkettigen oder verzweigten Rest mit 2 bis 8 C-Atomen steht,
- $CH_2O$-Benzoyl, wobei der aromatische Ring auch auch ein- bis dreifach mit einem geradkettigen oder verzweigten $C_1$-$C_4$-Alkyl-Rest, $OCH_3$, F oder Cl substituiert sein kann,
- $CH_2OCHO$,
- $CH_2OC(O)OCH_2Ph$,
- $CH_2NH$-Acyl, wobei Acyl für einen geradkettigen oder verzweigten Rest mit 2 bis 8 C-Atomen steht,
- $CH_2NH$-Benzoyl, wobei der aromatische Ring auch ein- bis dreifach mit einem geradkettigen oder verzweigten $C_1$-$C_4$-Alkyl-Rest, $OCH_3$, F oder Cl substituiert sein kann,
- $CH_2N(CH_2Ph)_2$, wobei die aromatischen Ringe auch ein- bis dreifach mit einem geradkettigem oder verzweigten $C_1$-$C_4$-Alkyl-Rest, $OCH_3$, F oder Cl substituiert sein können,
- $CH_2O$-$CH_2$-$CH$=$CH_2$,
- $CO_2H$,
- $CO_2M$, wobei M ein

Alkalimetall oder Erdalkalimetall bedeutet,
$CO_2N(X^4)_4$, wobei $X^4$ gleich oder verschieden ist und ein H-Atom oder einen geradkettigen oder verzweigten Alkylrest mit 1 bis 8 C-Atomen bedeutet,
$CO_2X^5$, wobei $X^5$
Methyl,
Ethyl,
Alkyl oder
Benzyl bedeutet;

$X^2$, $X^3$ für gleiche oder verschiedene Reste aus der Gruppe
H,
OH,
OTHP,
OAc,
$SiMe_3$,
$OSiMe_2t$-Bu,
$SiPh_2t$-Bu,
$OCH_2Ph$, wobei die Phenylgruppe auch ein- bis dreifach mit einem geradkettigen oder verzweigten $C_1$-$C_4$-Alkyl-Rest, $OCH_3$, F oder Cl substituiert sein kann,
O-$CH_2CH$=$CH_2$,
$OC(O)CH_2Ph$, wobei die Phenylgruppe auch ein- bis dreifach mit einem geradkettigen oder verzweigten $C_1$-$C_4$-Alkyl-Rest, $OCH_3$, F oder Cl substituiert sein kann,
OC(O)Ph, wobei die Phenylgruppe auch ein- bis dreifach mit einem geradkettigen oder verzweigten $C_1$-$C_4$-Alkyl-Rest, $OCH_3$, F oder Cl substituiert sein kann,
oder
OC(O)O-Alkyl steht,
wobei der Rest $X^2$ in α- oder β-Stellung, der Rest $X^3$ nur in α-Stellung vorkommt;

Y OH,
einen geradkettigen oder verzweigten Alkoxyrest mit 1 bis 8 C-Atomen,
O-$CH_2Ph$, wobei die Phenylgruppe auch ein- bis dreifach mit einem geradkettigen oder verzweigten $C_1$-$C_4$-Alkyl-Rest, $OCH_3$, F oder Cl substituiert sein kann,
O-Ph, wobei die Phenylgruppe auch ein- bis dreifach mit einem geradkettigen oder verzweigten $C_1$-$C_4$-Alkyl-Rest, $OCH_3$, F oder Cl substituiert sein kann,
$OM^1$, wobei $M^1$ für ein Alkalimetall steht,
$ONX^6_4$, wobei $X^6$
ein H-Atom oder
eine $C_1$-$C_8$-Alkylgruppe bedeutet,
OTHP,
$OCH_2$-CH=$CH_2$,
$NH_2$,
$NH(CH_2)_2SO_3H$,
$N(CH_3)(CH_2)_2SO_3H$,
$NHCH_2CO_2H$ oder $N(CH_3)CH_2CO_2H$ bedeutet;
die Hydroxylgruppe in der Ringposition C-3 sowohl in α- als auch in β-Stellung stehen kann und n gleich 0 bis 47 ist.

Bevorzugt sind Linker-modifizierte Gallensäurederivate der Formel I, wobei

$X^1$ $CH_2OH$,
$CH_2NH_2$,
$CH_2OSiMe_2t$-Bu,
$CH_2OTHP$,
$CO_2H$ oder
$CO_2CH_3$ bedeutet;

$X^2$, $X^3$ für gleiche oder verschiedene Reste ausgewählt aus der Gruppe bestehend aus
H,
OH,
OAc und
OTHP steht,
wobei der Rest $X^2$ in α- oder β-Stellung, der Rest $X^3$ nur in α-Stellung vorkommt;

Y OH,
$OCH_3$ oder
Ot-Bu bedeutet;
die Hydroxylgruppe in der Ringposition C-3 vorzugsweise in α-Stellung steht und n gleich 0 bis 47 ist.

Die erfindungsgemäßen, Linker-modifizierten Gallensäurederivate der Formel I sind alle stereoisomeren Formen und physiologisch verträglichen Salze dieser Verbindungen.

Unter dem Begriff THP wird eine Tetrahydropyranyl-Gruppe verstanden. Die Begriffe Me, t-Bu und Ac bedeuten Methyl, tertiär-Butyl beziehungsweise Acetyl. Trityl steht für Triphenylmethyl.

Die Erfindung betrifft ferner ein Verfahren zur Herstellung der erfindungsgemäßen Verbindungen, dadurch gekennzeichnet, daß prinzipiell entsprechend Schema 1 ein geeignetes Gallensäureketon II, welches entweder aus der Literatur bekannt ist, käuflich erworben wird oder gemäß Schema 2 hergestellt wird und wobei $X^2$, $X^3$ und Y Reste wie Formel I bedeuten, mit einem geeigneten Acetylenderivat III, welches aus der Literatur bekannt ist oder gemäß Schema 3 hergestellt wird und wobei $X^1$ ein Rest wie Formel I und n gleich null bis 47 ist, zu einem Additionsprodukt IV verknüpft wird, wobei $X^1$, $X^2$, $X^3$ und Y Reste wie in Formel I bedeuten und n gleich null bis 47 ist. Bei dieser Verknüpfungsreaktion wurde das Acetylenderivat III mit einer starken Base wie z.B. einer Alkyllithiumverbindung zuvor in das entsprechende Lithiumderivat umgewandelt. Das primäre Additionsprodukt IV, in dem die Reste $X^1$, $X^2$, $X^3$ und Y Reste wie in Formel I sind und n gleich null bis 47 ist, wird durch katalytische Hydrierung in die entsprechende gesättigte Verbindung I und gegebenenfalls durch weitere Umwandlungen wie Abspaltung von Schutzgruppen, Umwandlung funktioneller Gruppen in weitere erfindungsgemäße Verbindungen der Formel I überführt, worin $X^1$, $X^2$, $X^3$ und Y Reste wir in Formel I sind und n gleich null bis 47 ist. Bei dieser Reaktionsfolge müssen die erfindungsgemäßen Reste wie unten exemplarisch

gezeigt wird (Schema 2) so gewählt sein, daß keine störenden Nebenreaktionen auftreten.

Schema 1

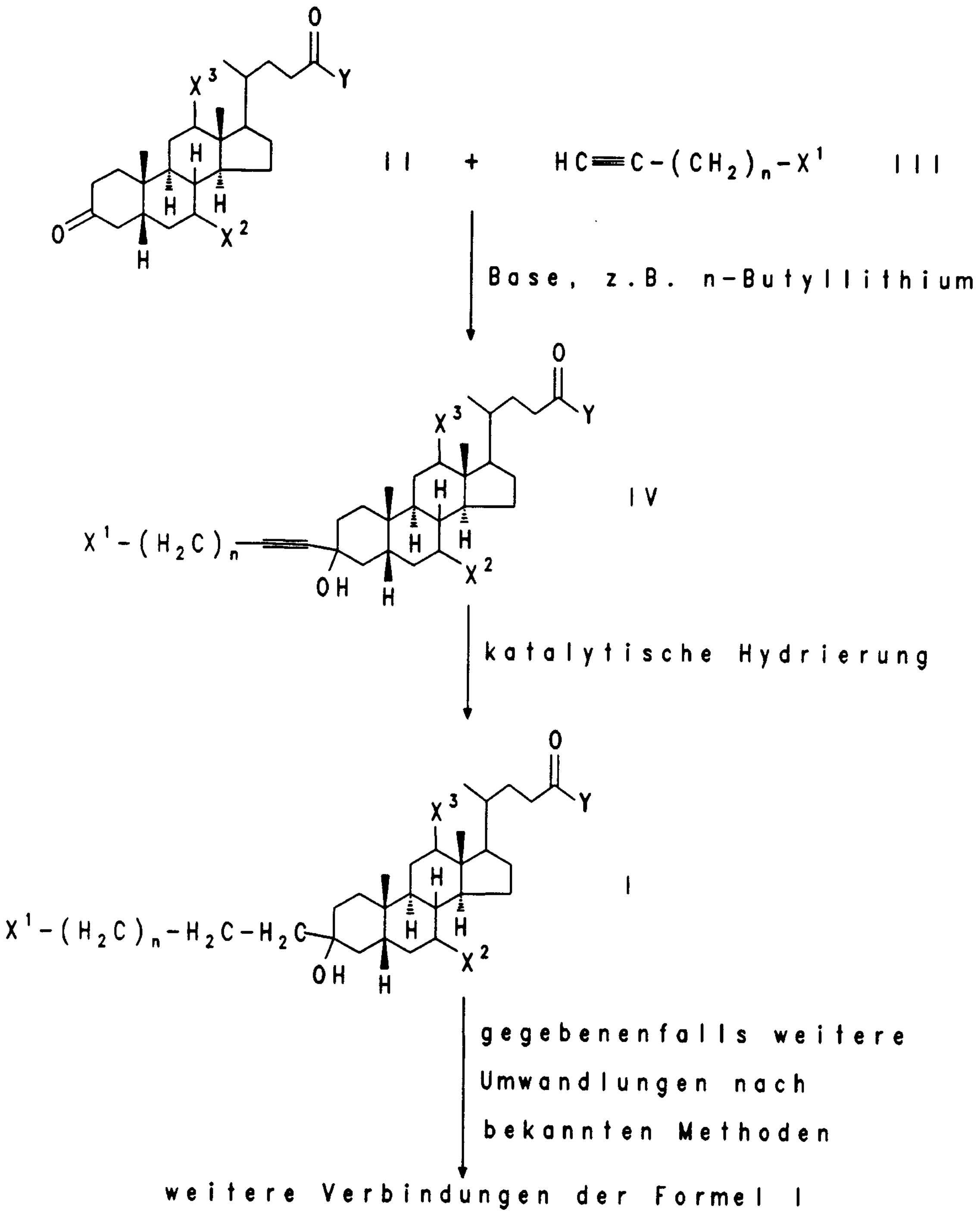


Schema 2 zeigt exemplarisch die Herstellung von erfindungsgemäßen Verbindungen für n = 3; $X^2 = X^3$ = OTHP, OH; $X^1$ = $CH_2OH$, $CH_2NH_2$, $CO_2H$. In den Reaktionsfolgen ausgehend von Verbindung I (e) wird gezeigt, wie eine verbindungsgemäße Verbindung der Formel I in andere erfindungsgemäße Verbindungen der Formel I umgewandelt werden kann.

Bei der Umsetzung des THP geschützten 3-Keto-cholsäuremethylester (Verbindung IIc) mit 5-Hexin-1-ol wurde die Alkoholgruppe nicht geschützt, sondern durch ein zweites Äquivalent n-Butyllithium intermediär in das Lithiumalkoholat

überführt. Bei langkettigen Alkinolen n ≧ 6 wurden bevorzugt die OH-Gruppen als t-Butyldimethylsilylether geschützt eingesetzt.

Überraschend wurde gefunden, daß langkettige Alkinole bzw. die entsprechenden t-Butyldimethylsilylether mit besonders hoher Ausbeute an Ketone addieren, wenn das Lithiumacetylid mit n-Butyllithium bei erhöhter Temperatur, vorzugsweise unter Rückfluß generiert wird. In Schema 2 bedeutet:

p-TosH p-Toluolsulfonsäure
THF Tetrahydrofuran

Schema 2

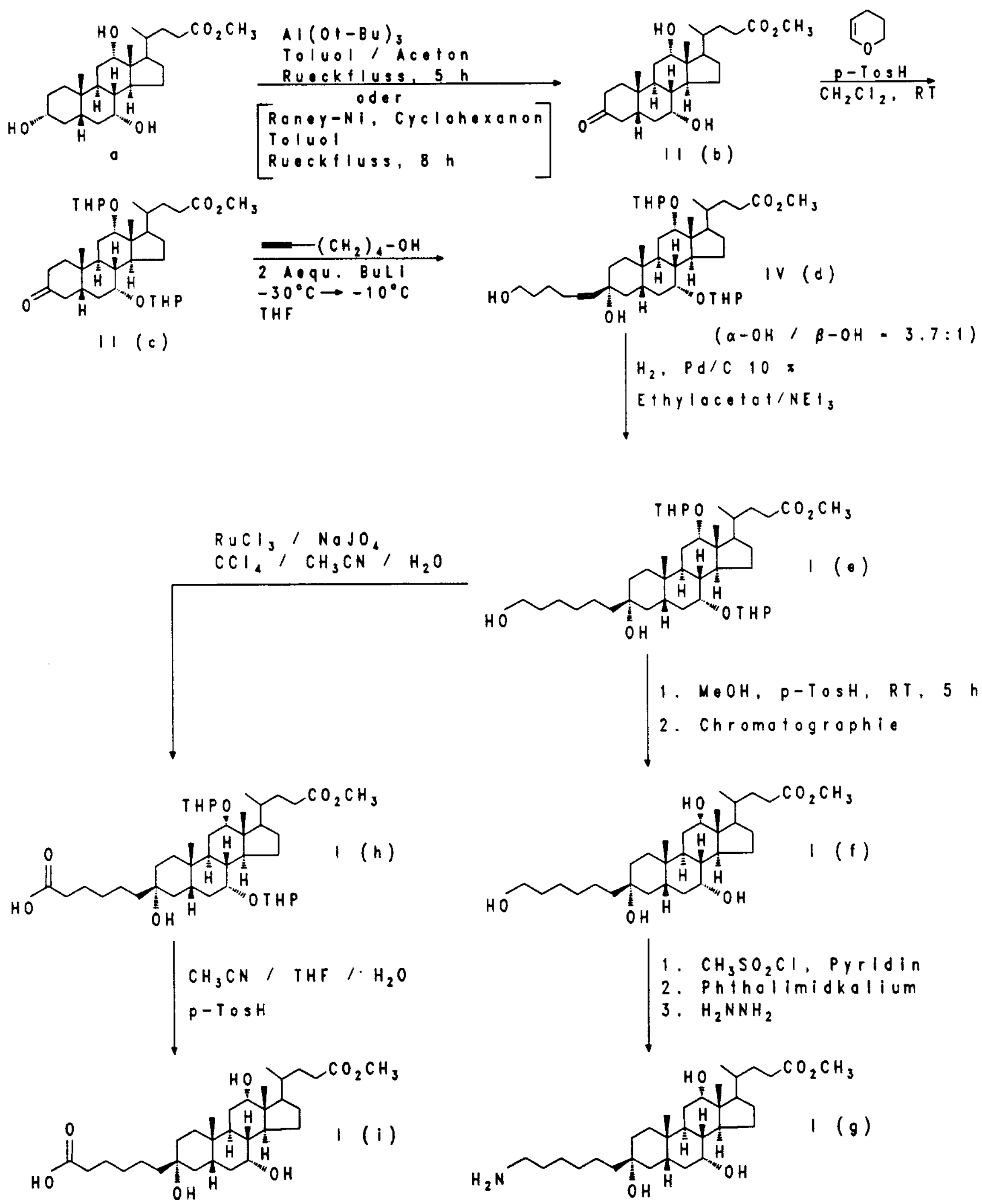


Soweit die Alkinole nicht käuflich zu erwerben sind, erfolgt die Herstellung in Anlehnung an Literaturverfahren entsprechend Schema 3. Dieses Verfahren ist für Alkinole, die bei dem erfindungsgemäßen Verfahren eingesetzt werden und die jeweils erforderliche Kettenlänge haben, anwendbar. Schlüsselschritt ist die Isomerisierung einer Dreifachbindung

in die ω-terminale Position unter den angegebenen Reaktionsbedingungen.

Schema 3

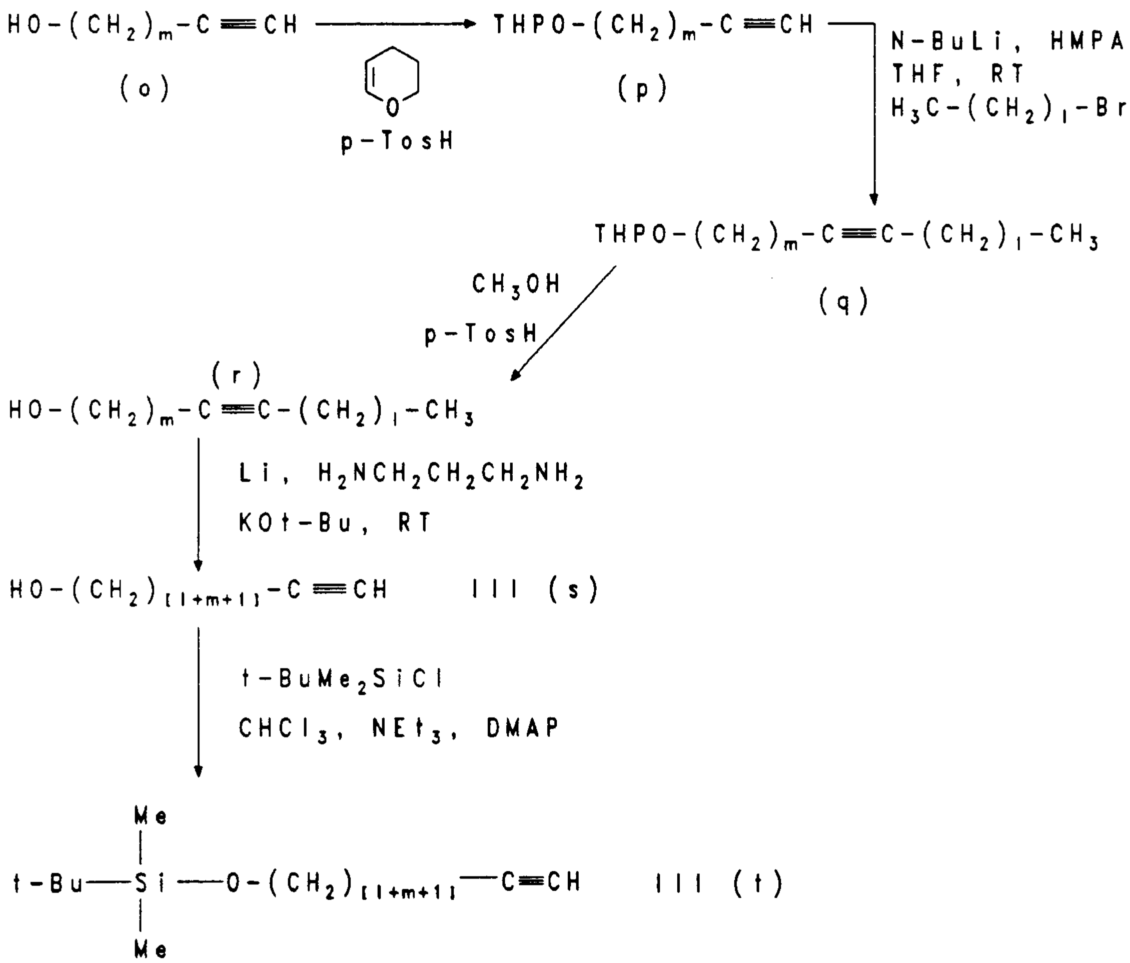


In Schema 3 bedeutet:

HMPA Hexamethylphosphorsäuretriamid
DMAP 4-Dimethylaminopyridin

Es gilt außerdem, daß n = l + m ist.

Die erfindungsgemäßen Verbindungen der Formel I besitzen wertvolle pharmakologische Eigenschaften. Die Erfindung betrifft daher auch Arzneimittel, enthaltend mindestens eine erfindungsgemäße Verbindung der Formel I und die Verwendung der Verbindungen der Formel I als Arzneimittel, insbesondere zur Senkung eines erhöhten Lipidspiegels.

Zur Herstellung eines Arzneimittels enthaltend mindestens eine Verbindung der Formel I werden die Verbindungen der allgemeinen Formel I gelöst oder suspendiert in pharmakologisch unbedenklichen organischen Lösungsmitteln, wie ein- oder mehrwertigen Alkoholen, wie z.B. Ethanol oder Glycerin, in Triacetin, Ölen wie z.B. Sonnenblumenöl, Lebertran, Ethern, wie z.B. Diethylenglykoldimethylether oder auch Polyethern, wie z.B. Polyethylenglykol, oder auch in Gegenwart anderer pharmakologisch unbedenklicher Polymerträger, wie z.B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffen gegeben werden.

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch im Dosisbereich 10 bis 1000 mg.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvisikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml T61®, einer wäßrigen Lösung von 2,5 mg Tetracain HCl, 100 m Embutramid und 25 mg Mebezoniumjodid getötet. Der Dünndarm wurde entnommen und mit eiskalter physiologischer Kochsalzlösung gesprült. Die terminalen 7/10 des Dünndarms (gemessen in oral rectaler Richtung, d.h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethylsulfonylfluorid/1 mg/l Trypsin Inhibitor v. Sojabohnen (32 (U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, Deutschland) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wird der Niederschlag verworfen und der Überstand, der die Bürstensaummembranen enthält, 30 Minuten bei 48000 x g (20000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0°C wurde erneut 15 Minuten bei 3000 x g zentrifugiert. Der Überstand wurde anschließend nochmals 30 Minuten bei 48000 x g (20000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol), /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm ∅, Millipore, Eschborn, Deutschland) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes, (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, Deutschland) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, Deutschland) aufgelöst und die Radioaktivität durch Flüssigkeits-Szintillationsmessung in einem Meßgerät TriCarb 2500 (Canberra Packard GmbH, Frankfurt, Deutschland) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle - gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 2 zusammengefaßt.

Tabelle 2 zeigt Meßwerte der Hemmung der [$^3$H]-Taurocholataufnahme in Bürsensaummembranveseikel des Ileums von Kanninchen. Angegeben sind die Quotienten ausden $IC_{50Na}$-Werten der Referenzsubstanz als Taurochenodesoxycholat (TCDC) und der jeweiligen Testsubstanz.

Tabelle 2:

| Verbindungen aus Beispiel | $IC_{50Na}$-TCDC [µmol] / $IC_{50Na}$-Substanz [µmol] |
|---|---|
| 36 (α-Isomer) | 0.13 |
| 36 (ß-Isomer) | 0.13 |
| 41 | 0.16 |
| 45 | 0.12 |

1. Herstellung von Gallensäureketonen II

Beispiel 1: Cholsäure-t-butylester

100 g (0.2448 mol) Cholsäure wurden in 500 ml Tetrahydrofuran (THF) vorgelegt. Bei 0°-5°C tropfte man 345.8 ml (2.448 mol) Trifluoressigsäureanhydrid Zu. Man ließ 1 h bei Raumtemperatur rühren. Anschließend kühlte man auf 0°C und tropfte 459.4 ml (4.90 mol) t-Butanol bei 0°-5°C zu. Man ließ über Nacht bei Raumtemperatur rühren. Bei 0°C tropfte man 500 ml 25 %ige wäßrige Ammoniumhydroxidlösung zu und ließ 5 h bei Raumtemperatur rühren. Das Reaktionsgemisch wurde auf Wasser gegossen und mit Ethylacetat extrahiert (3x). Trocknen der vereinigten organischen Phasen($Na_2SO_4$), Entfernen des Lösungsmittels (Ethylacetat) und Chromatographie auf Kieselgel (Ethylacetat:MeOH = 9:1) ergab 55 g (48 % der Theorie) Cholsäure-t-butylester.
$C_{28}H_{48}O_5$ (464.7) MS (FAB) 471 M+Li

Beispiel 2: 3-Keto-cholsäure-t-butylester

41 g (0.088 mol) Cholsäure-t-butylester aus Beispiel 1 wurden in 600 ml Toluol/300 ml Aceton in Gegenwart von 43.5 g (0.177 mol) Aluminium-t-butylat 5 h unter Rückfluß erhitzt. Das Reaktionsgemisch wurde auf ein Gemisch aus 2 l 1 molarer Schwefelsäure und 1 kg Eis gegossen und mit Ethylacetat extrahiert (3x). Trocknen der vereinigten organischen Phasen mit $MgSO_4$, Entfernen des Lösungsmittels und Flash-Chromatographie auf Kieselgel (Ethylacetat/Cyclohexan = 2:1) ergab 29.5 g (72 % der Theorie) 3-Keto-cholsäure-t-butylester.
$C_{28}H_{46}O_5$ (462.7) MS (FAB) 469 M+Li

Beispiel 3: 3-Keto-7,12-ditetrahydropyranyl-cholsäure-t-butylester

1.0 g (0.0021 mol) 3-Keto-cholsäure-t-butylester aus Beispiel 2 wurde in 25 ml Dichlormethan gelöst. Man gab 2.5 ml Dihydropyran zu und setzte eine katalytische Menge (1 Kristall) p-Toluolsulfonsäuremonohydrat zu. Nach 3 h bei Raumtemperatur wurde das Reaktionsgemisch auf gesättigte wäßrige Natriumchloridlösung gegossen und mit Dichlormethan extrahiert (3x). Die vereinigten organischen Phasen wurden getrocknet ($Na_2SO_4$) und vom Lösungsmittel befreit. Flash-Chromatographie auf Kieselgel (Ethylacetat/Cyclohexan = 1:4) ergab 3-Keto-7,12-ditetrahydropyranyl-

cholsäure-t-butylester in quantiativer Ausbeute.
$C_{38}H_{62}O_7$ (630.9) MS (FAB) 637 M+Li

Beispiel 4: 3-Keto-cholsäuremethylester

In Analogie zu Beispiel 2 wurde aus Cholsäuremethylester 3-Keto-cholsäuremethylester gewonnen.
$C_{25}H_{40}O_5$ (420.5) MS (FAB) 427 M+Li

Beispiel 5: 3-Keto-7,12-ditetrahydropyranylcholsäuremethylester

In Analogie zu Beispiel 3 wurde aus 3-Keto-cholsäuremethylester 3-Keto-7,12-ditetrahydropyranylcholsäuremethylester gewonnen.
$C_{35}H_{56}O_7$ (588.8) MS (FAB) 595 M+Li

Beispiel 6: 3-Keto-7,12-diacetylcholsäuremethylester

35 g (0.0832 mol) 3-Keto-cholsäuremethylester wurden in 200 ml Pyridin gelöst und mit 75 ml Acetanhydrid versetzt. Man erhitzte 2 h unter Rückfluß. Nach dem Erkalten wurde das Reaktionsgemisch auf 4 l Wasser getropft. Das Produkt wurde abgesaugt, mit Wasser gewaschen und mit 200 ml Diisopropylether ausgerührt.
Ausbeute: 35 g (83 % der Theorie) 3-Keto-7,12-diacetylcholsäuremethylester.
$C_{29}H_{44}O_7$ (504.7) MS (FAB) 511 M+Li

2. Herstellung von Acetylenderivaten III

Beispiel 7: 11-Tetrahydropyranyloxyundecin

17.5 g (0.104 mol) 10-Undecin-1-ol wurden in 100 ml Dichlormethan (25 ml Dihydropyran gelöst und mit einer katalytischen Menge (Spatelspitze) p-Toluolsulfonsäuremonohydrat versetzt. Man ließ über Nacht bei Raumtemperatur stehen und goß das Reaktionsgemisch auf gesättigte wäßrige Natriumchlorid-Lösung. Extraktion mit Dichlormethan (3x) und Trocknen ($Na_2SO_4$) der vereinigten organischen Phasen ergab nach Flash-Chromatographie auf Kieselgel (n-Heptan/Ethylacetat = 10:1) 24 g (92 % der Theorie) 11-Tetrahydropyranyloxyundecin.
$C_{16}H_{24}O_2$ (252.4) MS (DCl) 253 M +H

Beispiel 8: 1-Tetrahydropyranyloxy-10-hentriacontin

5 g (0.0198 mol) 11-Tetrahydropyranyloxyundecin aus Beispiel 7 wurden unter Argonatmosphäre in einem Gemisch aus 50 ml THF und 25 ml Hexamethylphosphorsäuretriamid vorgelegt. Bei -60°C wurden 12.4 ml (0.0198 mol) n-Butyllithium in Hexan zugetropft. Nach 30 min bei -60°C wurden 9.0 g (0.0248 mol) 1-Bromeicosan gelöst in 25 ml THF bei -60°C zugetropft. Man ließ auf Raumtemperatur kommen. Nach 18 h bei Raumtemperatur wurde der Ansatz auf 25 %ige wäßrige $NaH_2PO_4$-Lösung gegossen und mit n-Heptan extrahiert (3x). Die vereinigten organischen Phasenwurden getrocknet ($Na_2SO_4$) und vom Lösungsmittel befreit. Flash-Chromatographie auf Kieselgel (n-Heptan/Ethylacetat = 15:1) ergab 6.5 g (61.3 % der Theorie) 1-Tetrahydiopyranyloxy-10-hentriacontin.
$C_{36}H_{68}O_2$ (532,9) MS (DCl) 533 M+H

Beispiel 9: Hentriacont-10-in-1-ol

25.6 g (0.048 mol) 1-Tetrahydropyranyloxy-10-hentriacotin aus Beispiel 8 wurden in einem Gemisch aus 100 ml THF und 50 ml Methanol gelöst und bei Raumtemperatur mit einer katalytischen Menge (Spatelspitze) p-Toluolsulfonsäuremonohydrat versetzt. Nach 3 h bei Raumtemperatur wurde das Reaktionsgemisch auf 1.5 l Wasser gegossen und 30 min gerührt. Das Produkt wurde abgesaugt, mit Wasser gewaschen und im Hochvakuum getrocknet.
Ausbeute 20.8 g (96 % der Theorie) Hentriacon-10-in-1-ol. $C_{31}H_{60}O$ (448.8) MS (DCl) 449 M+H

Beispiel 10: Hentriacont-30-in-1-ol

Unter Argonatmosphäre wurde bei Raumtemperatur 1.0 g (0.1448 mol) Lithiumdraht stückchenweise zu 75 ml Propylendiamin gegeben. Man rührte 15 min bei Raumtemperatur und erhitzte anschließend auf 70°C bis zum Verschwinden der Blaufärbung. Man kühlte auf Raumtemperatur und gab 10 g (0.0891 mol) Kalium-t-butylat zu, ließ 15 min bei Raumtemperatur rühren und gab 10 g (0.0223 mol) Hentriacont-10-in-1-ol aus Beispiel 9 als Feststoff zu. Man ließ über

Nacht bei Raumtemperatur stehen, goß den Ansatz auf Eiswasser und ließ 30 min bei Raumtemperatur rühren. Das Produkt wurde abgesaugt und im Hochvakuum getrocknet.
Ausbeute: 9.6 g (96 % der Theorie) Hentriacont-30-in-1-ol.
$C_{31}H_{60}O$ (448.8) MS (DCI) 449 M+H

Beispiel 11: 1-t-Butyldimethylsilyloxy-hentriacont-30-in

9.6 g (0.0214 mol) Hentriacont-30-in-1-ol aus Beispiel 10 wurden in einem Gemisch aus 100 ml Chloroform und 4.4 ml Triethylamin 1 h intensiv bei Raumtemperatur gerührt. Man gab 3.5 g (0.0235 mol) t-Butyldimethylsilylchlorid zu und anschließend eine katalytische Menge (Spatelspitze) 4-Dimethylaminopyridin. Man ließ über Nacht bei Raumtemperatur stehen und zog die Hälfte des Lösungsmittels ab. Die verbleibende Lösung wurde über eine Kieselgelsäule (Chloroform) chromatographiert.
Ausbeute: 8.7 g (73 % der Theorie) 1-t-Butyldimethylsilyloxy-hentriacont-30-in. $C_{37}H_{74}OSi$ (563.1) MS (DCI) 448 M - $Me_2Si$ t-Bu + H; MS(EI) 563 M+H

Beispiel 12: 1-t-Butyldimethylsilyloxy-10-undecin

In Analogie zu Beispiel 11 wurde das 1-t-Butyldimethylsilyloxy-hentriacont-30-in gewonnen.
$C_{17}H_{34}OSi$ (282.5) MS (EI) 283 M+H

3. Umsetzung von Acetylenderivaten III mit Ketonen II zu den Additionsprodukten IV

Beispiel 13

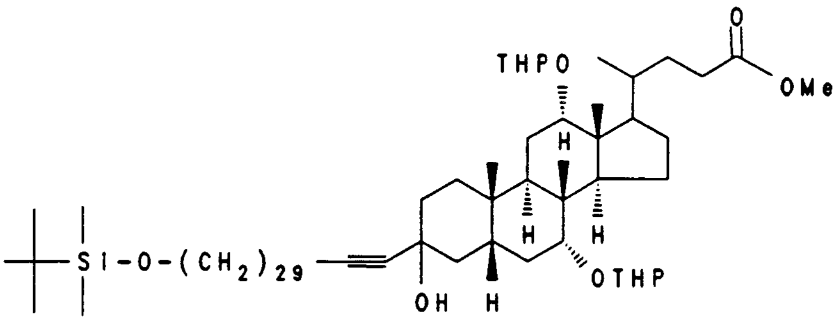

10.0 g (0.0178 mol) 1-t-Butyldimethylsilyloxy-hentriacont-30-in aus Beispiel 11 wurden unter Argonatmosphäre in 100 ml THF vorgelegt. Bei Raumtemperatur tropfte man 11.1 ml (0.0178 mol) n-Butyllithium in Hexan zu. Man erhitzte 1 h unter Rückfluß. Bei Raumtemperatur wurden 5.23 g (0.0089 mol) 3-Keto-7,12-ditetrahydropyranylcholsäuremethylester aus Beispiel 5, gelöst in 10 ml THF zugetropft. Man ließ 1 h bei Raumtemperatur rühren, goß das Reaktionsgemisch auf 25 %ige wäßrige Natriumdihydrogenphosphatlösung und extrahierte mit Ethylacetat (3x). Trocknen ($Na_2SO_4$) der vereinigten Phasen, Abziehen des Lösungsmittels und Flash-Chromatographie auf Kieselgel (Ethylacetat/Cyclohexan = 1:9 → 1:5) ergab 2.0 g (20 %) vom 3β-OH-Isomeren und 6.5 g (64 %) vom 3α-OH-Isomeren. Gesamtausbeute: 8,5 g (84 % der Theorie)
$C_{72}H_{130}O_8$ Si (1151.9) MS (FAB) 1158 M+Li

Beispiel 14

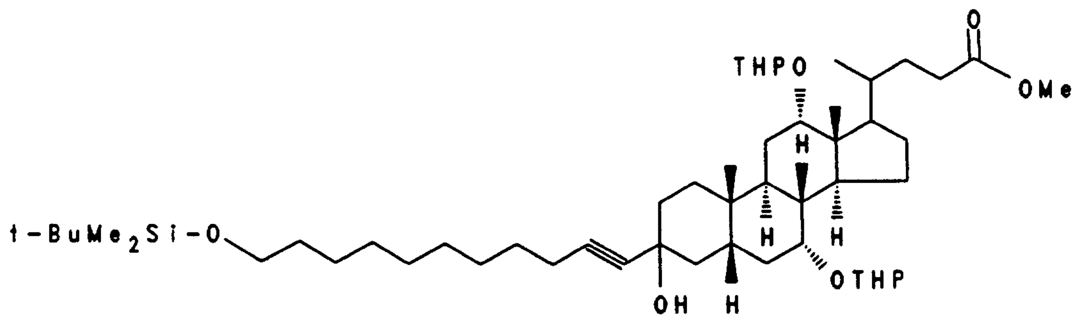

In Analogie zu Beispiel 13 wurde die oben angegebene Verbindung erhalten. Ausbeute ausgehend von 5.0 g 3-Keto-7,12-ditetrahydropyranylcholsäuremethylester aus Beispiel 5: 1.0 g (9 %) 3β-OH-Isomer und 8.4 g (72 %) 3α-OH-Isomer. Im Unterschied zur Herstellung der in Beispiel 13 erhaltenen Verbindung wurde das n-Butyllithium bei -60°C zugetropft und der 3-Keto-7,12-ditetrahydropyranylcholsäuremethylester aus Beispiel 5 bei -60°C zugegeben. Die Reaktion erfolgte innerhalb 3 h zwischen -60°C und -10°C. $C_{52}H_{30}O_8Si$ (871.4) MS (FAB) 877 M+Li

Beispiel 15

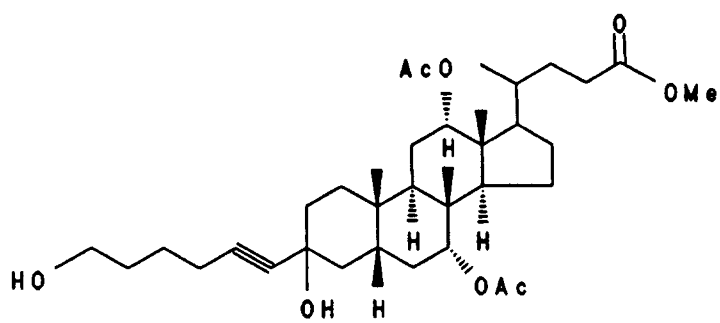

In Analogie zu Beispiel 13 und 14 wurde die oben angegebene Verbindung erhalten. Aus 15.0 g 3-Keto-7,12-diacetylcholsäuremethylester aus Beispiel 6 erhielt man mit 3 Äquivalenten 5-Hexin-1-ol 3.0 g (17 %) 3β-OH-Isomer und 10.1 g (56 %) 3α-OH-Isomer.
Gesamtausbeute: 13.1 g (73 % der Theorie) Im Unterschied zu Beispiel 13 wurden folgende Veränderungen durchgeführt: Es wurden 3 Äquivalente 5-Hexin-1-ol eingesetzt und entsprechende Äquivalente n-Butyllithium. Das n-Butyllithium wurde bei -30°C zugetropft. Nach Zugabe von 3-Keto-1,12-diacetylcholsäuremethylester aus Beispiel 6 bei -30°C erfolgte die Reaktion bei -30°C innerhalb von 4 h.
$C_{35}H_{54}O_8$ (602.8) MS (FAB) 609 M+Li

Beispiel 16

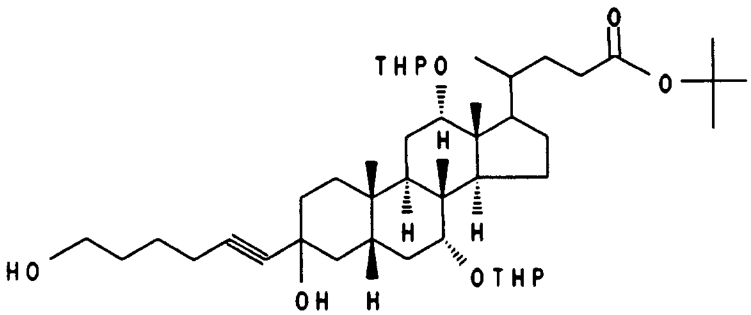

In Analogie zu Beispiel 15 wurde die oben angegebene Verbindung erhalten. Gesamtausbeute (3α-OH- und 3β-OH Isomer): 88 % der Theorie.
$C_{44}H_{72}O_8$ (729) MS (FAB) 735 M+Li

Beispiel 17

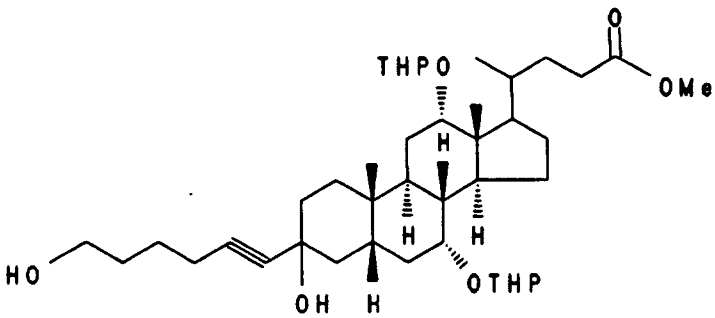

In Analogie zu Beispiel 16 wurde die oben angegebene Verbindung erhalten. Aus 12.0 g 3-Keto-7,12-ditetrahydropyranylcholsäuremethylester aus Beispiel 5 erhielt man die oben angegebene Verbindung in 77 %iger Ausbeute.
$C_{41}H_{66}O_8$ (687.0) MS(FAB) 693 M+Li

4. Umwandlung der Additionsprodukte IV in erfindungsgemäße Verbindungen I

Beispiel 18

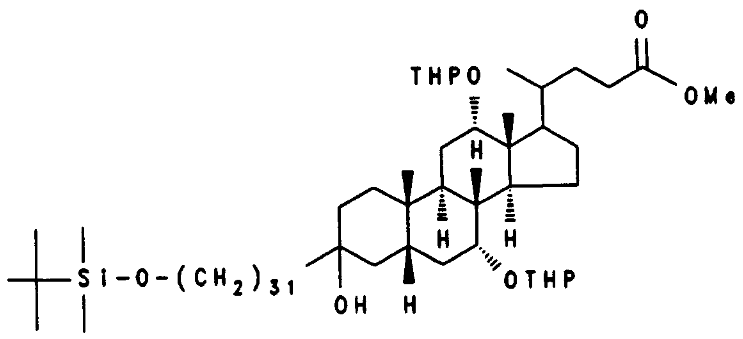

6.2 g (0.0054 mol) des 3α-OH-Isomeren der Verbindung aus Beispiel 13 wurden in 100 ml Ethylacetat (10 ml Triethylamin gelöst und in Gegenwart einer katalytischen Menge Pd/C (10 %) bei Raumtemperatur und Normaldruck hydriert. Nach dem Ende der Wasserstoffaufnahme setzte man 200 ml Chloroform zu und filtrierte den Katalysator ab. Abziehen des Lösungsmittels im Vakuum ergab die oben angegebene Verbindung in quantitativer Ausbeute $C_{72}H_{134}O_8Si$ (1155.9) MS (FAB) 1162 M+Li

In Analogie zu Beispiel 18 wurden die Beispiele der Tabelle 1 erhalten.

Tabelle 1

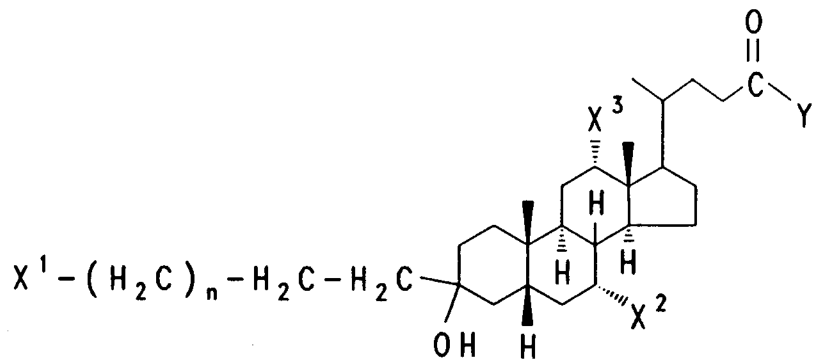

I

| Beispiel | $X^1$ | $X^2 = X^3$ | Y | n | Isomer | Ausgangs-material | Summenformel (Molmasse) | MS (FAB) |
|---|---|---|---|---|---|---|---|---|
| 19 | OSi t-Bu$Me_2$ | OTHP | $OCH_3$ | 29 | 3ß-OH | 13 | $C_{72}H_{134}O_8Si$ (1155.9) | 1162 M+Li |
| 20 | OSi t-Bu$Me_2$ | OTHP | $OCH_3$ | 9 | 3α-OH | 14 | $C_{52}H_{94}O_8Si$ (875.4) | 881 M+Li |
| 21 | OSi t-Bu$Me_2$ | OTHP | $OCH_3$ | 9 | 3ß-OH | 14 | $C_{52}H_{94}O_8Si$ (875.4) | 881 M+Li |
| 22 | OH | OAc | $OCH_3$ | 4 | 3α-OH | 15 | $C_{35}H_{58}O_8$ (606.8) | 613 M+Li |
| 23 | OH | OAc | $OCH_3$ | 4 | 3ß-OH | 15 | $C_{35}H_{58}O_8$ (606.8) | 613 M+Li |
| 24 | OH | OTHP | $OCH_3$ | 4 | 3α-OH | 17 | $C_{41}H_{70}O_8$ (691.0) | 697 M+Li |

| Beispiel | $X^1$ | $X^2 = X^3$ | Y | n | Isomer | Ausgangs-material | Summenformel (Molmasse) | MS (FAB) |
|---|---|---|---|---|---|---|---|---|
| 25 | OH | OTHP | $OCH_3$ | 4 | 3ß-OH | 17 | $C_{41}H_{70}O_8$ (691.0) | 697 M+Li |
| 26 | OH | OTHP | Ot-Bu | 4 | 3α-OH | 16 | $C_{44}H_{72}O_8$ (729.1) | 735 M+Li |
| 27 | OH | OTHP | Ot-Bu | 4 | 3ß-OH | 16 | $C_{44}H_{72}O_8$ (729.1) | 735 M+Li |

Beispiel 28

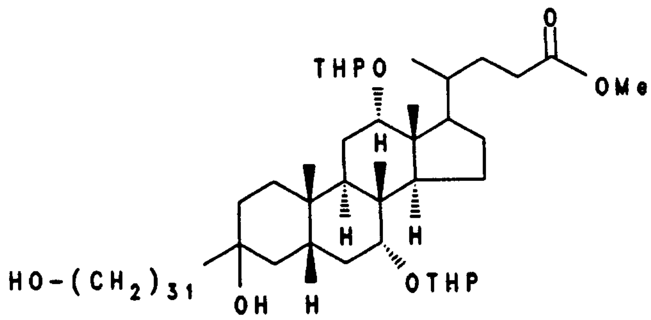

6.3 g der in Beispiel 18 erhaltenen Verbindung wurden in 75 ml THF gelöst und über Nacht bei Raumtemperatur mit 5.1 g (0.0161 mol) Tetrabutylammoniumfluorid-Trihydrat gerührt. Das Reaktionsgemisch wurde auf Wasser gegossen mit Chloroform extrahiert (3x) und die vereinigten organischen Phasen wurden getrocknet ($Na_2SO_4$). Entfernen des Lösungsmittels ergab die oben angegebene Verbindung in quantitativer Ausbeute.
$C_{66}H_{120}O_8$ (1041.7) MS (FAB) 1047 M+Li

Beispiel 29

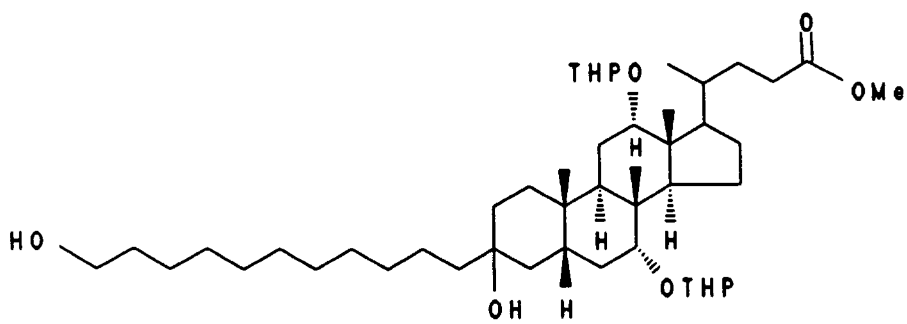

In Analogie zu Beispiel 28 wurde oben angegebene Verbindung ausgehend von der Verbindung aus Beispiel 20 erhalten.
$C_{46}H_{80}O_8$ MS (FAB) 767 M+Li

Beispiel 30

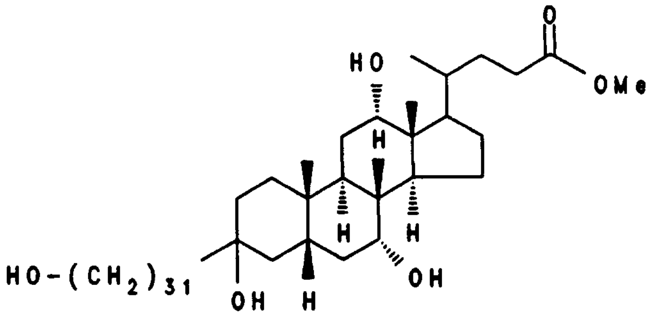

3.1 g der in Beispiel 28 erhalenen Verbindung wurden in einem Gemisch aus 10 ml Chloroform und 5 ml Methanol gelöst und mit einer katalytischen Menge (Spatelspitze) p-Toluolsulfonsäure-Monohydrat versetzt. Man ließ über Nacht bei Raumtemperatur stehen und zog das Lösungsmittel ab. Flash-Chromatographie auf Kieselgel (Chloroform/Methanol = 5:1) ergab 1.7 g (72 % der Theorie) der oben angegebenen Verbindung.
$C_{56}H_{104}O_6$ (873.4) MS(FAB) 879 M+Li

Beispiel 31

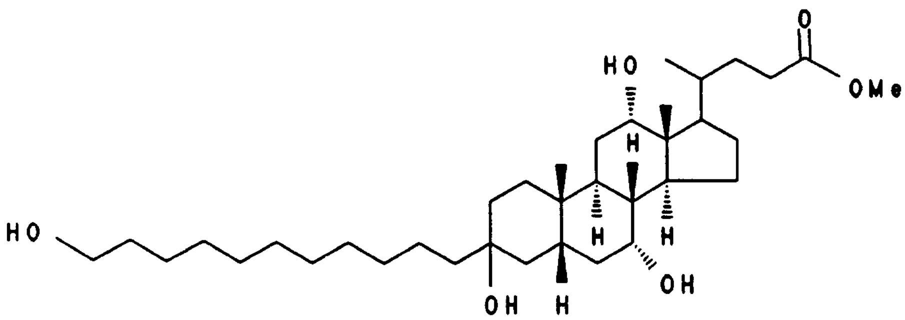

In Analogie zu Beispiel 30 erhielt man die oben angegebene Verbindung ausgehend von der Verbindung aus Beispiel 29.
$C_{36}H_{64}O_6$ (592.9) MS (FAB) 599 M+Li

Beispiel 32/1

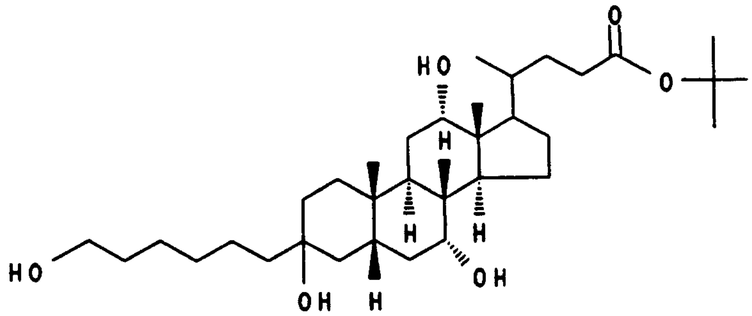

1.4 g (0.0019 mol) der Verbindungen, die in den Beispielen 26 und 24 als 3α-OH/3β-OH Isomerengemisch erhalten wurden, wurden in 25 ml Methanol gelöst und bei Raumtemperatur mit 50 mg p-Toluolsulfonsäure-Monohydrat versetzt. Nach 5 h bei Raumtemperatur wurde der Ansatz auf gesättigte wäßrige $NaHCO_3$-Lösung gegossen. Extraktion mit Ethylacetat (3x), Trocknen der vereinigten Phasen ($Na_2SO_4$), Abziehen des Lösungsmittels und Säulenchromatographie auf Kieselgel (Ethylacetat → Ethylacetat/Methanol = 9:1) ergab 165 mg (15 %) 3β-OH Isomeres und 684 mg (63 %) 3α-OH Isomeres. Gesamtausbeute: 78 % der Theorie an oben angegebener Verbindung.
$C_{34}H_{60}O_6$ (564.8) MS (FAB) 571 M+Li

Beispiel 32/2

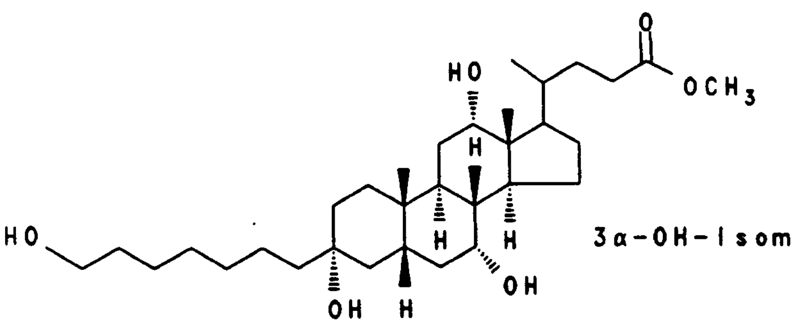

3α-OH-Isomeres

In Analogie zu Beispiel 32/1 wurde die oben angegebene Verbindung und das entsprechende 3β-OH-Isomere ausgehend von den Verbindungen aus Beispiel 22/23 gewonnen. Aus 8.7 g Ausgangsmaterial erhielt man 1.01 g (14 %) an 3β-OH Isomerem und 4.26 g (57 %) an 3α-OH-Isomerem. Gesamtausbeute: 71 % der Theorie an oben angegebener Verbindung.
$C_{31}H_{54}O_6$ (522.7) MS (ES+) 529 M+Li

Beispiel 33

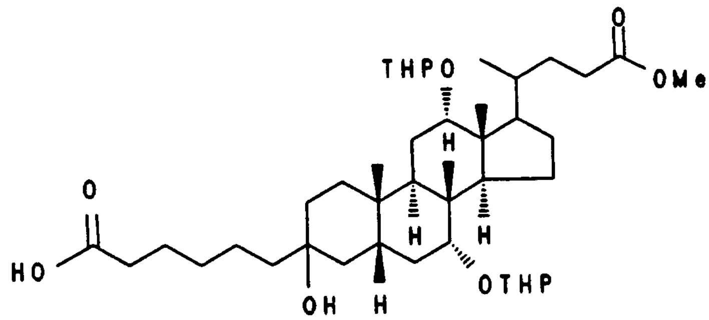

9.2 g (0.0133 mol) eines Isomerengemischs aus den in den Beispielen 24 und 25 erhaltenen Verbindungen wurden bei Raumtemperatur in einem Gemisch aus 75 ml Tetrachlorkohlenstoff, 75 ml Acetonitril und 100 ml Wasser vorgelegt und mit 14.2 g (0.0666 mol) Natriumperjodat und 100 mg Ruthenium(III)-chlorid (als Katalysator) versetzt und 3 h bei Raumtemperatur gerührt. Das Reaktionsgemisch wurde mit Methylenchlorid 3x extrahiert und die vereinigten organischen Phasen getrocknet. Abziehen des Lösungsmittels im Vakuum und Flash Chromatographie auf Kieselgel (Ethylacetat/Cyclohexan = 1:1) ergab 7.5 g (80 % der Theorie) der oben angegebenen Verbindung als Gemisch der 3α-OH und 3β-OH Isomeren.

$C_{41}H_{68}O_9$ (705.0) MS (FAB) 711 M+Li

In Analogie zu Beispiel 33 wurden die folgenden Beispiele erhalten:

Beispiel 34

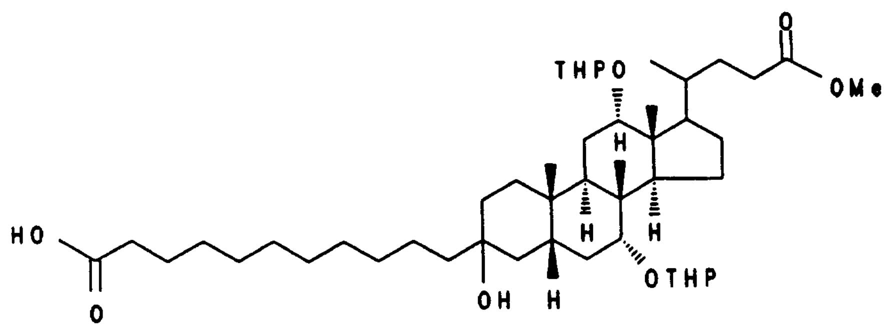

$C_{46}H_{78}O_9$ (775.1) MS (FAB) 781 M+Li

Beispiel 35

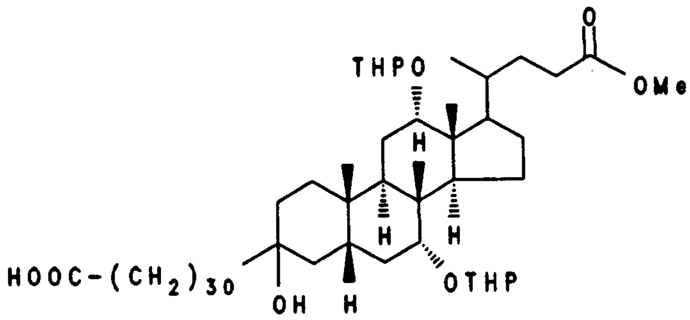

$C_{66}H_{118}O_9$ (1055.7) MS (FAB) 1062 M+Li

Beispiel 36

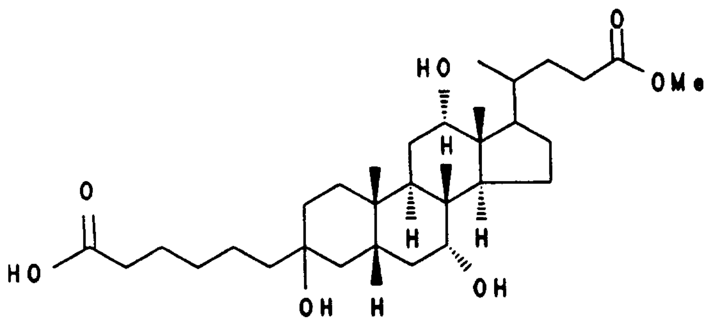

800 mg (0.00114 mol) der nach Beispiel 33 als Isomerengemisch gewonnenen Verbindung wurden in einem Gemisch aus 5 ml THF, 15 ml Acetonitril und 5 ml Wasser vorgelegt und mit einer katalytischen Menge (Spatelspitze) p-Toluolsulfonsäuremonohydrat versetzt. Nach 16 h bei Raumtemperatur wurde das Reaktionsgemisch auf Wasser gegossen und mit Methylenchlorid 3x extrahiert. Die vereinigten organischen Phasen wurden getrocknet ($Na_2SO_4$) und vom Lösungsmittel befreit. Flash-Chromatographie auf Kieselgel (Ethylacetat → Ethylacetat/MeOH = 9:1) ergab 110 mg (18 %) 3β-OH-Isomeres und 357 mg (59 %) 3α-OH-Isomeres. Gesamtausbeute: 467 mg (77 % der Theorie) der oben angegebenen Verbindung.

$C_{31}H_{52}O_7$ (536.7) MS (FAB) 543 M+Li, 549 M+2Li-H

Beispiel 37

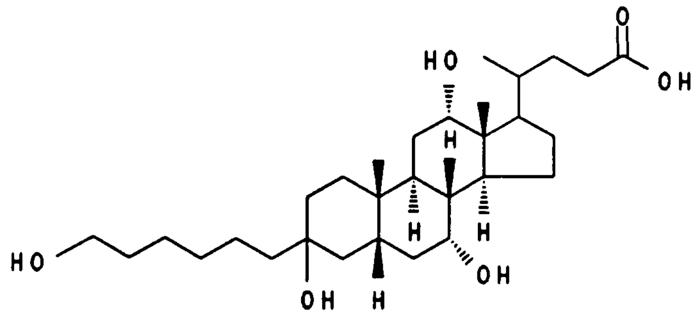

5.0 g (0.0082 mol) des Isomerengemisches, welches nach den Beispielen 22 und 23 erhalten wurde, wurden in 50 ml Ethanol gelöst und mit 100 ml molarer wäßriger Natronlauge über Nacht bei Raumtemperatur gerührt. Das Reaktionsgemisch wurde auf 25 %ige wäßrige $NaH_2PO_4$ gegossen und mit Ethylacetat 3x extrahiert. Die vereinigten organischen Phasen wurden getrocknet ($Na_2SO_4$). Abziehen des Lösungsmittels ergab 4.1 g (98 % der Theorie) der freien Säure der oben angegebenen Formel.
$C_{30}H_{52}O_6$ (508.7) MS (ES+) 515 M+Li

Beispiel 38

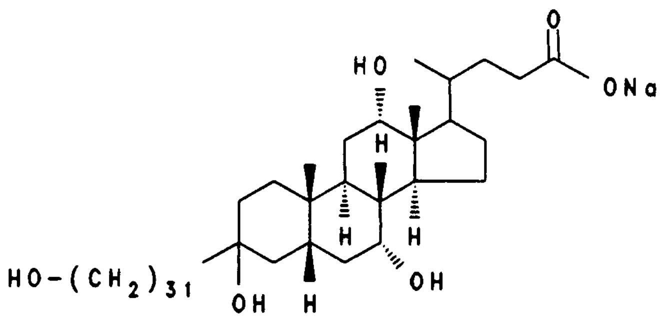

In Analogie zu Beispiel 37 wurde oben angegebene Verbindung ausgehend von der in Beispiel 30 gewonnenen Verbindung erhalten. Abweichend von der in Beispiel 37 angegebenen Vorschrift wurde in Beispiel 38 ein Gemisch von THF und Methanol (2:1) als Lösungsmittel verwendet, wobei das Natriumsalz auskristallisierte.
Ausbeute: 270 mg (89 % der Theorie) ausgehend von 300 mg der in Beispiel 30 erhaltenen Verbindung $C_{55}H_{101}O_6Na$ (881.4) MS (FAB) 881 M+H, 903 M+Na
Die freie Säure kann durch Ansäuern mit HCl erhalten werden.

Beispiel 39

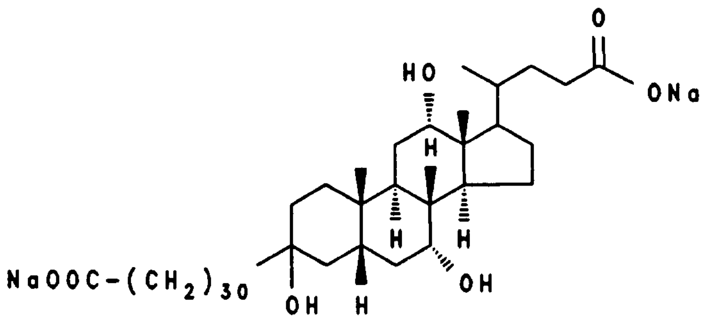

1.5 g (1.4 mmol) der Verbindung, welche in Beispiel 35 erhalten wurde, wurden in einem Gemisch aus 40 ml THF und 20 ml Methanol mit einer katalytischen Menge (Spatelspitze) p-Toluolsulfonsäure versetzt und 18 h bei Raumtemperatur gerührt. Anschließend gab man 5 ml 2N wäßrige Natronlauge zu und ließ über Nacht rühren. Dabei fiel das Di-Natriumsalz aus, welches abgesaugt und mit Methanol und Diisopropylether gewaschen wurde.
Ausbeute: 800 mg (61 % der Theorie) der oben angegebenen Verbindung.
$C_{55}H_{98}O_7Na_2$ (917.4) MS (FAB) 917 (M+H), 939 (M+Na)
Die freie Säure kann man durch Ansäuern mit verdünnter Salzsäure erhalten.

Beispiel 40

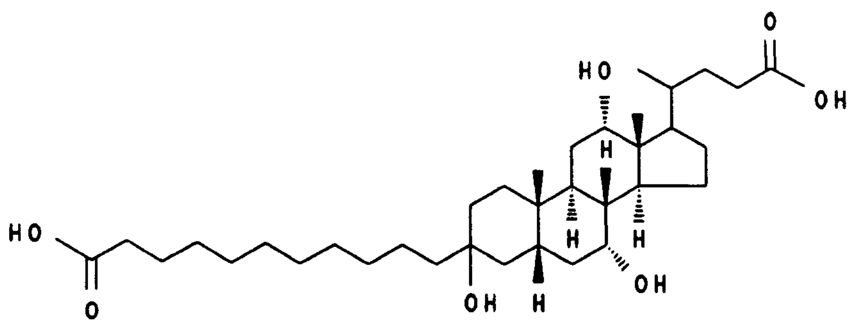

500 mg (0.65 mmol) der nach Beispiel 34 erhaltenen Verbindung wurden in 20 ml Methanol unter Zusatz einer Spatelspitze p-Toluolsulfonsäure-Monohydrat 5 h bei Raumtemperatur gerührt. Anschließend gab man 3.2 ml 1N wäßrige Natronlauge zu und ließ über Nacht stehen. Das Reaktionsgemisch wurde auf 10 %ige wäßrige $NaH_2PO_4$-Lösung gegossen und 30 min gerührt. Das Produkt wurde abgesaugt, mit Wasser gewaschen und im Hochvakuum getrocknet.
Ausbeute: 360 mg (94 % der Theorie) der oben angegebenen Verbindung.
$C_{35}H_{60}O_7$ (592.9) MS (FAB) 615 M+Na-H

Beispiel 41

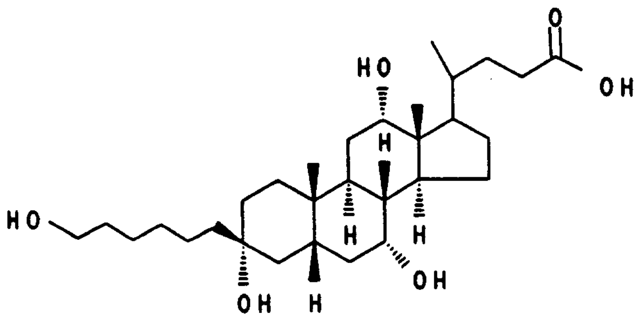

5 g (7.71 mmol) des nach Beispiel 22 erhaltenen 3α-OH-Isomeren wurden in 100 ml Ethanol gelöst und bei Raumtemperatur mit 200 ml 5 molarer wäßriger Natronlauge versetzt. Nach 18 h wurde das Reaktionsgemisch auf 500 ml 25 %ige wäßrige $NaH_2PO_4$-Lösung gegossen. Extraktion mit Ethylacetat (3x), Trocknen ($Na_2SO_4$) der vereinigten organischen Phasen und Entfernen des Lösungsmittels ergab eine quantitative Ausbeute von 3.95 g an oben angegebener Verbindung.

$C_{30}H_{52}O_6$ (508.6) MS (FAB) 515 M+Li, 521 M+2Li-H

Entsprechend wurde bei der Herstellung des 3β-OH-Isomeren verfahren.

Beispiel 42

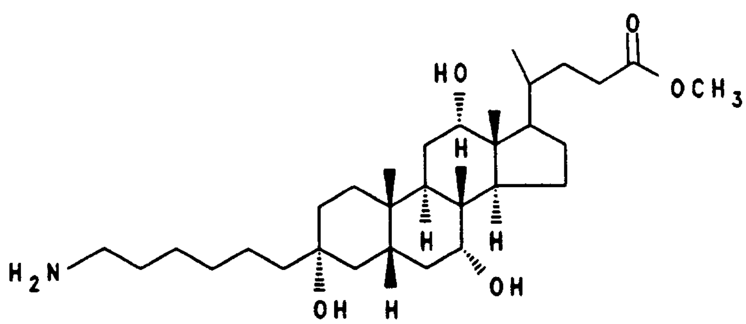

4.2 g (8.04 mmol) des nach Beispiel 32/2 erhaltenen reinen 3α-OH-Isomeren wurden in 25 ml Pyridin vorgelegt. Bei 0°C tropfte man 775 µl (9.81 mmol) Methansulfonsäurechlorid zu und ließ 2 h bei 0°C rühren. Das Reaktionsgemisch wurde auf Eiswasser gegossen und mit Ethylacetat 3x extrahiert. Die vereinigten organischen Phasen wurden getrocknet ($Na_2SO_4$) und eingeengt. Es ergab sich eine quantitative Ausbeute von 4.8 g bezogen auf das Mesylat von Beispiel 32/2.

Dieses Material (4.8 g) wurde in 50 ml DMF in Gegenwart von 6.3 g Phthalimidkalium 2.5 h bei 80°C gerührt. Nach dem Abkühlen wurde auf Wasser gegossen und mit Ethylacetat 3x extrahiert. Trocknen der vereinigten organischen Phasen ($Na_2SO_4$), Abziehen des Lösungsmittels und Chromatographie auf Kieselgel (Ethylacetat) ergab 5.02 g (96 % der Theorie) des entsprechenden Phthalimidderivates.

4.9 g (7.52 mmol) des Phthalimidderivates wurden in 100 ml Methanol gelöst. Bei Raumtemperatur wurden 10.7 ml Hydrazinhydrat zugetropft. Nach 1.5 h Rühren bei Raumtemperatur wurde mit 2N wäßriger Salzsäure angesäuert und mit gesättigter wäßriger $NaHCO_3$-Lösung neutralisiert. Dreimalige Extraktion mit Ethylacetat, Trocknen der vereinigten organischen Phasen ($Na_2SO_4$) und Abziehen des Lösungsmittels ergab nach Ausrühren des Rückstandes (mit Ethylacetat/n-Heptan = 2:1), Absaugen und Trocknen im Hochvakuum 3.78 g (96 % der Theorie) der oben angegebenen Verbindung.

$C_{31}H_{55}NO_5$ (521.7) MS (FAB) 528 M+Li

Beispiel 43

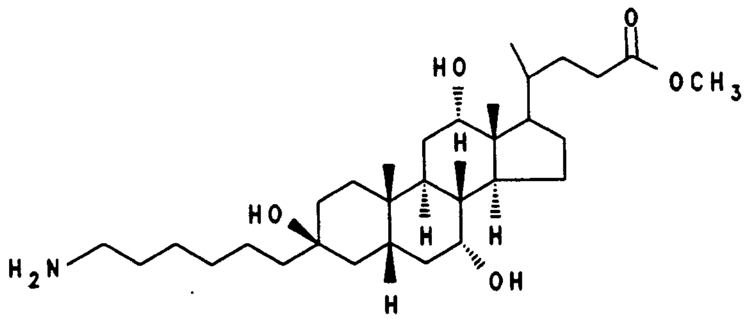

Ausgehend von dem nach Beispiel 32/2 erhaltenen reinen 3β-OH-Isomeren wurde in Analogie zu Beispiel 42 das 3β-OH-Isomere, wie oben angegeben, gewonnen.
$C_{31}H_{55}NO_5$ (521.7) MS (FAB) 522 M+H, 528 M+Li

Beispiel 44

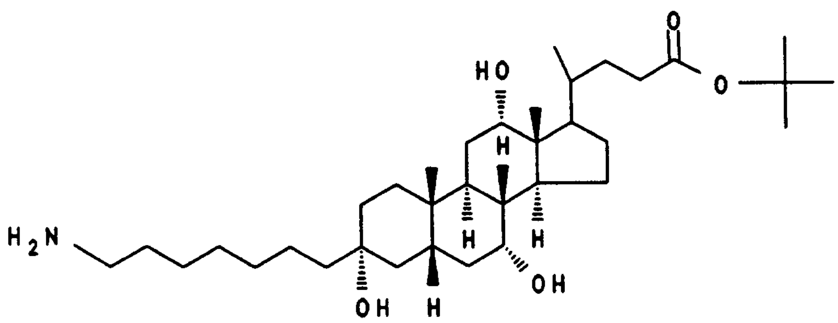

Ausgehend von dem nach Beispiel 32/1 gewonnenen reinen 3α-OH-Isomeren wurde in Analogie zu Beispiel 42 das 3α-OH-Isomere, wie oben angegeben, erhalten.
$C_{34}H_{61}NO_5$ (563.8) MS (FAB) 570 M+Li

Beispiel 45

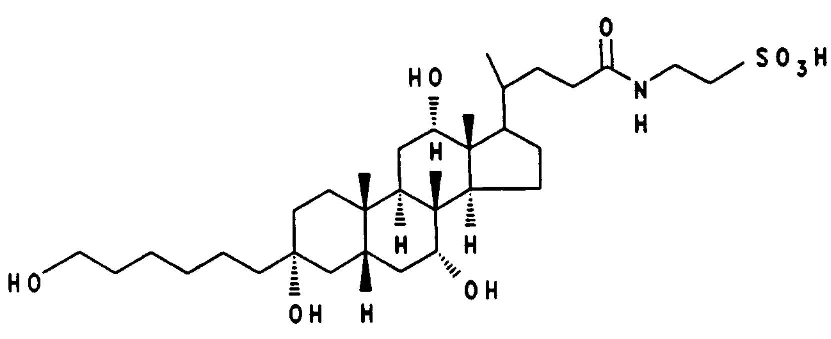

102 mg (0.2 mmol) des nach Beispiel 41 erhaltenen 3α-OH-Isomeren wurden in einem Gemisch aus 5 ml THF und 0.5

ml $NEt_3$ bei 0°C vorgelegt. Es wurden 30 µl (0.3 mmol) Chlorameisensäurethylester zugetropft und 15 min bei 0°C gerührt. Anschließend gab man 88 mg (0.7 mmol) Taurin gelöst in 0.1 N wäßriger Natronlauge zu und ließ 1 h bei Raumtemperatur rühren. Man gab wäßrige $NaH_2PO_4$-Lösung zu, trennte die Phasen und extrahierte die wäßrige Phase 2x mit THF. Die vereinigten organischen Phasen wurden getrocknet ($Na_2SO_4$). Entfernen des Lösungsmittels und Verreiben des Rückstandes mit Ethylacetat/n-Heptan ergab 113 mg (92 %) der oben angegebenen Verbindung.
$C_{32}H_{57}NO_8S$ (615.8) MS (ES-) 614 M-H

Beispiel 46

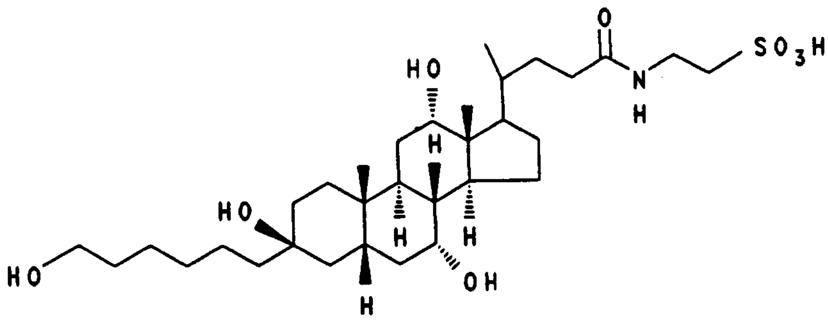

In Analogie zu Beispiel 45 wurde die oben angegebene Verbindung aus dem 3β-OH-Isomeren der nach Beispiel 41 erhaltenen Verbindung gewonnen.
$C_{32}H_{57}NO_8S$ (615.8) MS (ES-) 614 n-H

**Patentansprüche**

1. Linker-modifizierte Gallensäurederivate der Formel I,

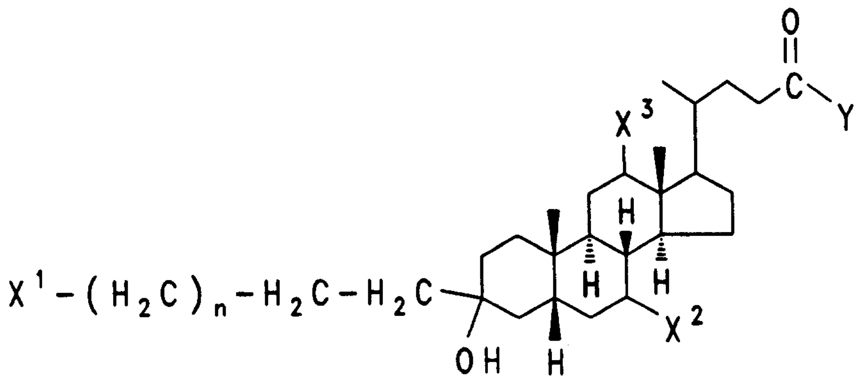

I

wobei

X1 $CH_2OH$,
$CH_2NH_2$,
$CH_2OSil$, wobei Sil eine Silylschutzgruppe der Formel
$SiMe_3$,
$SiMe_2t$-Bu oder
$SiPh_2t$-Bu bedeutet,
$CH_2OTHP$,
$CH_2OCH_2Ph$, wobei Phenyl p-Methoxy substituiert sein kann,
$CH_2O$-Trityl,
$CH_2O$-Acyl, wobei Acyl für einen geradkettigen oder verzweigten Rest mit 2 bis 8 C-Atomen steht,

$CH_2O$-Benzoyl, wobei der aromatische Ring auch ein- bis dreifach mit einem geradkettigen oder verzweigten $C_1$-$C_4$-Alkyl-Rest, $OCH_3$, F oder Cl substituiert sein kann,
$CH_2OCHO$,
$CH_2OC(O)OCH_2Ph$,
$CH_2NH$-Acyl, wobei Acyl für einen geradkettigen oder verzweigten Rest mit 2-8 C-Atomen steht,
$CH_2NH$-Benzoyl, wobei der aromatische Ring auch ein- bis dreifach mit einem geradkettigen oder verzweigten $C_1$-$C_4$-Alkyl-Rest, $OCH_3$, F oder Cl substituiert sein kann,
$CH_2N(CH_2Ph)_2$, wobei die aromatischen Ringe auch ein- bis dreifach mit einem geradkettigen oder verzweigten $C_1$-$C_4$-Alkyl-Rest, $OCH_3$, F oder Cl substituiert sein können,
$CH_2O$-$CH_2$-$CH$=$CH_2$,
$CO_2H$,
$CO_2M$, wobei M ein
Alkalimetall oder Erdalkalimetall bedeutet,
$CO_2N(X^4)_4$, wobei $X^4$ gleich oder verschieden ist und ein H-Atom oder einen geradkettigen oder verzweigten Alkylrest mit 1 bis 8 C-Atomen bedeutet,
$CO_2X^5$, wobei $X^5$
Methyl,
Ethyl,
Alkyl oder
Benzyl bedeutet;

$X^2$, $X^3$ für gleiche oder verschiedene Reste aus der Gruppe
H,
OH,
OTHP,
OAc,
$SiMe_3$,
$OSiMe_2t$-Bu,
$SiPh_2t$-Bu,
$OCH_2Ph$, wobei die Phenylgruppe auch ein- bis dreifach mit einem geradkettigen oder verzweigten $C_1$-$C_4$-Alkyl-Rest, $OCH_3$, F oder Cl substituiert sein kann,
O-$CH_2CH$=$CH_2$,
$OC(O)CH_2Ph$, wobei die Phenylgruppe auch ein- bis dreifach mit einem geradkettigen oder verzweigten $C_1$-$C_4$-Alkyl-Rest, $OCH_3$, F oder Cl substituiert sein kann,
OC(O)Ph, wobei die Phenylgruppe auch ein- bis dreifach mit einem geradkettigen oder verzweigten $C_1$-$C_4$-Alkyl-Rest, $OCH_3$, F oder Cl substituiert sein kann,
oder
OC(O)O-Alkyl steht,
wobei der Rest $X^2$ in $\alpha$- oder $\beta$-Stellung, der Rest $X^3$ nur in $\alpha$-Stellung vorkommt;

Y OH,
einen geradkettigen oder verzweigten Alkoxyrest mit 1 bis 8 C-Atomen,
O-$CH_2Ph$, wobei die Phenylgruppe auch ein- bis dreifach mit einem geradkettigen oder verzweigten $C_1$-$C_4$-Alkyl-Rest, $OCH_3$, F oder Cl substituiert sein kann,
O-Ph, wobei die Phenylgruppe auch ein- bis dreifach mit einem geradkettigen oder verzweigten $C_1$-$C_4$-Alkyl-Rest, $OCH_3$, F oder Cl substituiert sein kann,
$OM^1$, wobei $M^1$ für ein Alkalimetall steht,
$ONX^6_4$, wobei $X^6$
ein H-Atom oder
eine $C_1$-$C_8$-Alkylgruppe bedeutet,
OTHP,
$OCH_2$-$CH$=$CH_2$,
$NH_2$,
$NH(CH_2)_2SO_3H$,
$N(CH_3)(CH_2)_2SO_3H$,
$NHCH_2CO_2H$ oder $N(CH_3)CH_2CO_2N$ bedeutet;
die Hydroxylgruppe in der Ringposition C-3 sowohl in $\alpha$- als auch in $\beta$-Stellung stehen kann und n gleich 0 bis 47 ist.

2. Gallensäurederivate der Formel I gemäß Anspruch 1, wobei

| | |
|---|---|
| $X^1$ | $CH_2OH$,<br>$CH_2NH_2$<br>$CH_2OSiMe_2t\text{-}Bu$,<br>$CH_2OTHP$,<br>$CO_2H$ oder<br>$CO_2CH_3$ bedeutet; |
| $X^2$, $X^3$ | für gleiche oder verschiedene Reste ausgewählt aus der Gruppe bestehend aus<br>H,<br>OH,<br>OAc und<br>OTHP steht,<br>wobei der Rest $X^2$ in $\alpha$- oder $\beta$-Stellung der Rest $X^3$ nur in $\alpha$-Stellung vorkommt; |
| Y | OH,<br>$OCH_3$ oder<br>Ot-Bu bedeutet;<br>die Hydroxylgruppe in der Ringposition C-3 vorzugsweise in $\alpha$-Stellung steht und n gleich 0 bis 47 ist. |

3. Verfahren zur Herstellung von Gallensäurederivaten der Formel I gemäß Anspruch 1, dadurch gekennzeichnet, daß man ein geeignetes Gallensäureketon der Formel II

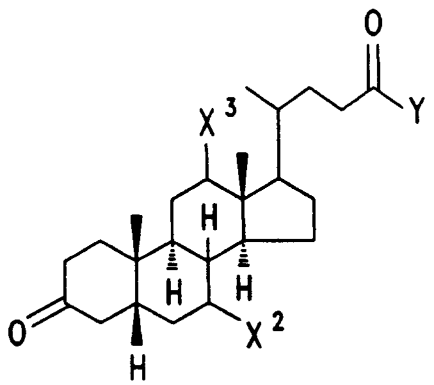

II

mit einer Verbindung der Formel III

$$HC{\equiv}C\text{-}(CH_2)_n\text{-}X^1 \text{ (III)} \qquad \text{(III)}$$

nach Überführung in das Acetylid durch eine starke Base zu einem Additionsprodukt der Formel IV

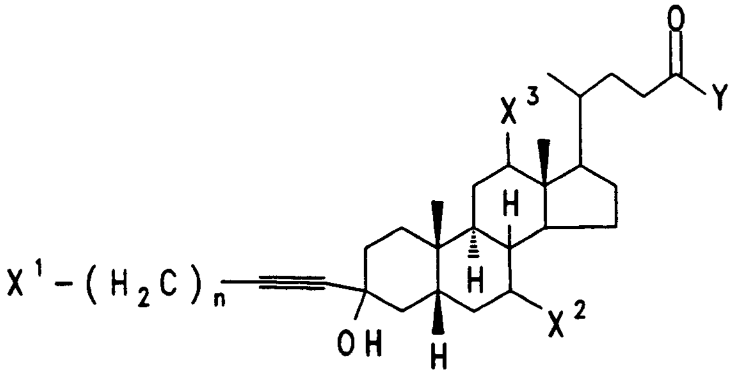

IV

umsetzt, welches dann durch eine Pd/C-katalysierte Hydrierung zu einer Verbindung der Formel I

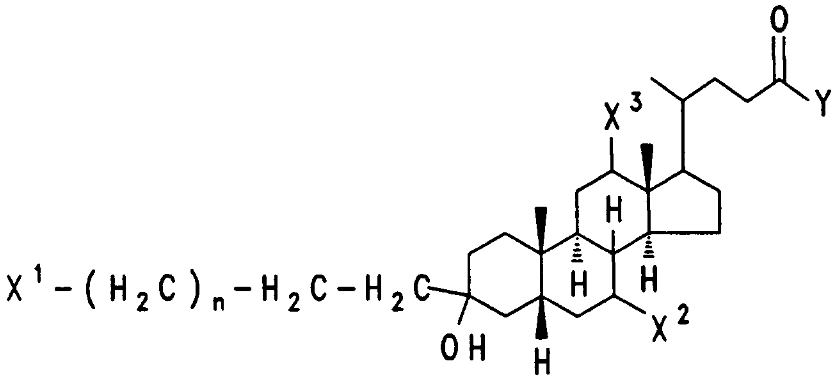

I

umsetzt wird und gegebenenfalls durch verschiedene, bekannte Methoden zu weiteren möglichen Verbindungen der Formel I umgesetzt werden kann, wobei in den Formeln die Reste $X^1$, $X^2$, $X^3$ und Y sowie n die in Anspruch 1 angegebenen Bedeutungen haben.

**4.** Arzneimittel enthaltend mindestens ein Gallensäurederivat der Formel I gemäß Anspruch 1.

**5.** Hypolipidaemikum enthaltend ein Gallensäurederivat der Formel I gemäß Anspruch 1.

**6.** Verwendung eines Gallensäurederivates der Formel I gemäß Anspruch 1 als Arzneimittel.